# EUROPEAN PATENT SPECIFICATION

(11) **EP 2 090 652 B1**
(45) Date of publication and mention of the grant of the patent: **19.10.2011**
(21) Application number: 07832500.8
(22) Date of filing: 26.11.2007
(51) Int. Cl.: C12N 15/00, C12N 15/09, C12Q 1/68, C12Q 1/70

(54) **METHOD FOR DETECTION OF HOP LATENT VIRUS, PRIMER SET FOR THE DETECTION, AND KIT FOR THE DETECTION**
VERFAHREN ZUM NACHWEIS VON LATENTEM HOPFENVIRUS, PRIMERSATZ FÜR DEN NACHWEIS UND KIT FÜR DEN NACHWEIS
PROCEDE DE DETECTION DU VIRUS LATENT DU HOUBLON, ENSEMBLE D'AMORCES ET NECESSAIRE DESTINES A CETTE DETECTION

(30) Priority: 30.11.2006 JP 2006324217
(43) Date of publication of application: 19.08.2009
(73) Proprietor: Sapporo Breweries Limited, Tokyo 150-8522 (JP)
(72) Inventor: OKADA, Yukio, Tokyo 150-8522 (JP); ITOGA, Yutaka, Tokyo 150-8522 (JP)
(74) Representative: Vossius & Partner
(86) International application number: PCT/JP2007/072775
(87) International publication number: WO 2008/066001

(56) References cited:
- EP-A2- 0 775 747
- JP-A- 09 173 100
- JP-A- 2004 089 180
- IMAI M ET AL: "Development of H5-RT-LAMP (loop-mediated isothermal amplification) system for rapid diagnosis of H5 avian influenza virus infection" VACCINE, ELSEVIER LTD, GB LNKD- DOI:10.1016/J.VACCINE.2006.05.046, vol. 24, no. 44-46, 10 November 2006 (2006-11-10), pages 6679-6682, XP025152002 ISSN: 0264-410X [retrieved on 2006-11-10]
- NOTOMI T ET AL: "Loop-mediated isothermal amplification of DNA" NUCLEIC ACIDS RESEARCH, OXFORD UNIVERSITY PRESS, SURREY, GB LNKD- DOI:10.1093/NAR/28.12.E63, vol. 28, no. 12, 1 December 2000 (2000-12-01), pages I-VII, XP007905272 ISSN: 0305-1048
- AOI Y ET AL: "Real-time quantitative LAMP (loop-mediated isothermal amplification of DNA) as a simple method for monitoring ammonia-oxidizing bacteria", JOURNAL OF BIOTECHNOLOGY, ELSEVIER SCIENCE PUBLISHERS, AMSTERDAM, NL, vol. 125, no. 4, 1 October 2006 (2006-10-01), pages 484-491, XP024956642, ISSN: 0168-1656, DOI: DOI:10.1016/J.JBIOTEC.2006.04.007 [retrieved on 2006-10-01]

### SEQUENCE LISTING

<110> Sapporo Breweries Ltd.
<120> Detection method of Hop latent virus, primer set and kit for detection of Hop latent virus
<130> FP07-0353-00
<150> JP 2006-324217 <151> 2006-11-30@
<160> 8
<170> PatentIn version 3. 1
<210> 1
   <211> 43
   <212> DNA
   <213> Artificial
<220>
   <223> primer
<400> 1
   gt aggat cag gcagcat gt c agaat gct gg gct agaaaca ggc 43
<210> 2
   <211> 43
   <212> DNA
   <213> Artificial
<220>
   <223> primer
<400> 2
   gt gt ct aaca at at ggct ac ct ccccaacc cct caagat c aac 43
<210> 3
   <211> 21
   <212> DNA
   <213> Artificial
<220>
   <223> primer
<400> 3
   agt t cctt gc t cgt agaagg g 21
<210> 4
   <211> 19
   <212> DNA
   <213> Artificial
<220>
   <223> primer
<400> 4
   gcacgt gct c agt aggcac 19
<210> 5
   <211> 19
   <212> DNA
   <213> Artificial
<220>
   <223> primer
<400> 5
   ggccagcgct caagcccac 19
<210> 6
   <211> 21
   <212> DNA
   <213> Artificial
<220>
   <223> primer
<400> 6
   gcagat cttc at cat at cct c 21
<210> 7
   <211> 243
   <212> DNA
   <213> Hop latent viroid
<400> 7
   agttccttgc tcgtagaagg gccgctatcc gcgtgactaa tgctgggcta gaaacaggcc     60
   ggccagcgct caagcccaca cctgacatgc tgcctgatcc taccaaccca tataacaaac    120
   cct cat t aga t gct t t gt t g at gat aaagc caagagt t gt gt ct aacaat at ggct acct 180
   ct gaggat at gat gaagat c t gcgt t gat c t t gaggggt t gggcgt gcct act gagcacg 240
   t gc 243
<210> 8
   <211> 185
   <212> DNA
   <213> Hop latent viroid
<400> 8
   aatgctgggc tagaaacagg ccggccagcg ctcaagccca cacctgacat gctgcctgat     60
   cct accaacc cat at aacaa accct cat t a gat gct t t gt t gat gat aaa gccaagagt t 120
   gt gt ct aaca at at ggct ac ct ct gaggat at gat gaaga t ct gcgt t ga t ct t gagggg 180
   t t ggg 185

## Description

### Technical Field

The present invention relates to a detection method, a detection primer set and a detection kit for hop latent virus.

### Background Art

Hop latent virus is a phytopathogenic virus belonging to the genus *Carlavirus,* and it primarily infects hops (*Humulus lupulus* L.). Once hops have been infected with hop latent virus, the infectious damage spreads throughout the hops via aphids.

In major hop-producing countries such as Germany, the United States, England and the Czech Republic, and in Japan, virus-free shoots produced by shoot tip culture are used for hop cultivation to prevent infectious damage by hop latent virus.

In hop cultivation farms, hop latent virus infection is periodically examined to halt secondary infection with hop latent virus. The examination of hop latent virus infection generally involves detection of hop-infecting hop latent virus by ELISA.

The detection method for hop latent virus by ELISA comprises a step of reacting hop latent virus particles in a specimen with a primary antibody that specifically recognizes hop latent virus on a microplate containing the primary antibody in solid phase, a step of washing off the components in the specimen that have not bound to the primary antibody, a step of reacting the hop latent virus particles that have bound to the primary antibody with a secondary antibody that is enzyme-labeled and specifically recognizes hop latent virus, a step of washing off the secondary antibody that has not bound to the hop latent virus particles, a step of performing enzyme reaction between the enzyme labeling the secondary antibody and a chemichromic or chemiluminescent substrate, and a step of estimating the amount of hop latent virus particles based on the intensity of coloring or luminescence obtained by the enzyme reaction.

[Non-patent document 1] Adams et al., 1982, Ann. Appl. Biol., Vol. 101, p.483-494
[Non-patent document 2] Hataya et al., 2000, Arch. Virol., Vol. 145, p.2503-2524
Previously, a method for detecting a hop latent virus by an RT-PCR method has been described using a primer that is designed based on an hop latent virus-derived gene, i.e. the hop latent virus coat protein gene (EP 0 775 747 A2). Imai et al., 2006, J. Vaccine, Vol. 24(44-46), p:6679-6682 describes Reverse Transcription-Loop-mediated isothermal AMPlification (RT-LAMP). Moreover, Aoi et al., Journal of Biotechnology, 2006, Vol. 125(4), p.484-491 describes real-time quantitative LAMP.

### Disclosure of the Invention

### Problems to be Solved by the Invention

When the titer or specificity of the hop latent virus-recognizing antibody used in ELISA is inadequate, it is affected by components in the specimen other than hop latent virus, and this may cause signal indicating the presence of hop latent virus to be evaluated as background signal, or nonspecific binding to be evaluated as signal indicating the presence of hop latent virus.

In addition, since detection methods for hop latent virus by ELISA comprise multiple steps as explained above, each of the steps must be carried out in the laboratory under the same conditions including reaction time and washing frequency, and if the number of specimens is increased the operation of each step becomes more complex and precise evaluation tends to be hindered.

Furthermore, because ELISA methods perform detection using viral particles themselves as the target, they cannot detect proviruses that are incorporated in viral genomes or host genomes, while hops infected with hop latent virus are often judged to be false-positive or undetectable in summer season in which higher temperature is known to notably reduce hop latent virus particle content.

In light of these problems, it is an object of the present invention to provide a method for detecting hop latent virus specifically and with high sensitivity in any season including during cultivation, harvesting and storage, regardless of the performance (titer and specificity) of the antibody used to detect the hop latent virus, the number of specimens and the location of evaluation. It is a further object of the invention to provide a detection primer set and detection kit for hop latent virus using the method.

### Means for Solving the Problems

In order to achieve the objects stated above, the invention provides a detection method for hop latent virus that comprises an extraction step in which hop latent virus RNA is extracted from a specimen, an amplification step in which a primer set containing 4 different oligonucleotides consisting of the nucleotide sequences represented by SEQ ID NO: 1-4 of the Sequence Listing is used for amplification of cDNA by Reverse Transcription-Loop-mediated isothermal AMPlification (RT-LAMP) using the RNA as template, and a judging step in which hop latent virus is judged to be present when amplification of cDNA containing the nucleotide sequence represented by SEQ ID NO: 8 of the Sequence Listing has occurred in the amplification step.

The present inventors have discovered that hop latent virus can be detected in a simple and highly sensitive manner by utilizing Reverse Transcription-Loop-mediated isothermal amplification (RT-LAMP) as an isothermal gene amplification reaction to amplify a specific region of the gene for the coat protein of hop latent virus. The term "RT-LAMP" refers to a method of amplifying cDNA complementary to an RNA template, incorporating reverse transcription reaction and isothermal gene amplification reaction by LAMP. The principle of this method is laid out in detail in International Patent Publication No. WO00/28082.

If RNA is extracted from tissue of hops for the purpose of determining the presence of hop latent virus infection, and a primer set containing 4 different oligonucleotides consisting of the nucleotide sequences represented by SEQ ID NO: 1-4 of the Sequence Listing is added to the extract for amplification of cDNA by RT-LAMP, according to the detection method described above, it is possible to detect cDNA of a partial sequence of genomic RNA of hop latent virus in a simple and highly sensitive manner, thus allowing the presence of hop latent virus infection to be determined.

The nucleotide sequence of genomic RNA of hop latent virus complementary to the nucleotide sequence represented by SEQ ID NO: 8 of the Sequence Listing, and the nucleotide sequences of genomic RNA of hop latent virus to which the 4 different oligonucleotides consisting of the nucleotide sequences represented by SEQ ID NO: 1-4 of the Sequence Listing anneal, are partial sequences of the gene for the coat protein of hop latent virus, and the regions of the genomic RNA of hop latent virus containing these nucleotide sequences are suitable target regions for amplification of cDNA by RT-LAMP. Consequently, the presence of hop latent virus can be specifically detected by confirming whether or not amplified cDNA is present.

The amplification step is preferably a step in which a primer set containing 6 different oligonucleotides consisting of the nucleotide sequences represented by SEQ ID NO: 1-6 of the Sequence Listing is used for amplification of cDNA by Reverse Transcription-Loop-mediated isothermal AMPlification (RT-LAMP) using the RNA as template.

If the cDNA is amplified with addition of 2 different oligonucleotides consisting of the nucleotide sequences represented by SEQ ID NO: 5 and 6 of the Sequence Listing to the 4 different oligonucleotides consisting of the nucleotide sequences represented by SEQ ID NO: 1-4 of the Sequence Listing, as Loop primers, the number of DNA synthesis origins will be increased, thus shortening the amplification time and allowing more efficient detection of hop latent virus.

The invention further provides a detection primer set for hop latent virus that comprises 4 different oligonucleotides consisting of the nucleotide sequences represented by SEQ ID NO: 1-4 of the Sequence Listing or 6 different oligonucleotides consisting of the nucleotide sequences represented by SEQ ID NO: 1-6 of the Sequence Listing.

If RNA is extracted from tissue of hops for the purpose of determining the presence of hop latent virus infection, and the aforementioned primer set is added to the extract for amplification of cDNA by RT-LAMP, it is possible to detect cDNA of a partial sequence of genomic RNA of hop latent virus in a simple and highly sensitive manner, thus allowing the presence of hop latent virus infection to be determined.

The invention still further provides a detection kit for hop latent virus which contains a detection primer set for hop latent virus comprising 4 different oligonucleotides consisting of the nucleotide sequences represented by SEQ ID NO: 1-4 of the Sequence Listing or 6 different oligonucleotides consisting of the nucleotide sequences represented by SEQ ID NO: 1-6 of the Sequence Listing, together with reverse transcriptase, strand displacing DNA synthase, dNTPs and buffer.

Using such a detection kit, it is possible to simultaneously procure the reagents necessary for detection of hop latent virus by RT-LAMP, and detection of hop latent virus can be easily accomplished without requiring complex procedures for preparation of reagent concentrations, even when the hop latent virus detection is in the field, for example.

### Effect of the Invention

According to the invention, it is possible to detect hop latent virus specifically and with high sensitivity in any season including during cultivation, harvesting and storage, regardless of the number of specimens or the location of evaluation. Moreover, the detection primer set and detection kit for hop latent virus according to the invention may be suitably used for RT-LAMP, thus allowing simple and high-sensitivity detection of hop latent virus.

### Brief Description of the Drawings

Fig. 1 is a diagram showing the positional relationship between the primer set comprising 4 different oligonucleotides consisting of the nucleotide sequences represented by SEQ ID NO: 1-4 of the Sequence Listing, and the target region of hop latent virus genomic RNA.
Fig. 2 is a gel diagram showing the band pattern obtained by using restriction enzyme NaeI for digestion of cDNA amplified by RT-LAMP using an RNA-diluted sample of a hop latent virus detection specimen, followed by polyacrylamide gel electrophoresis.

### Best Mode for Carrying Out the Invention

Preferred embodiments of the invention will now be described in detail.

The detection method for hop latent virus according to the invention is characterized by comprising an extraction step in which hop latent virus RNA is extracted from a specimen, an amplification step in which a primer set containing 4 different oligonucleotides consisting of the nucleotide sequences represented by SEQ ID NO: 1-4 of the Sequence Listing is used for amplification of cDNA by Reverse Transcription-Loop-mediated isothermal AMPlification (RT-LAMP) using the RNA as template, and a judging step in which hop latent virus is judged to be present when amplification of cDNA containing the nucleotide sequence represented by SEQ ID NO: 8 of the Sequence Listing has occurred in the amplification step.

"Hop latent virus" is an RNA virus belonging to the genus *Carlavirus.* The hop latent virus host is hops (*Humulus lupulus* L.), and hops infected with hop latent virus are known to have reduced cone yields.

The "specimen" used for the detection method described above may be, for example, plant leaves, stems, roots or fruit, although leaves and fruit are preferred for easier sampling, and leaves are more preferred. The sampling period for the specimen may be, for example, during cultivation, harvesting or storage.

In the extraction step, hop latent virus RNA present in plant cells surrounded by cell walls is extracted, and for example, the specimen may be physically homogenized in distilled water or buffer with a nearly alkaline pH, for extraction of the hop latent virus RNA.

The pH of the buffer is preferably about, for example, pH 8.0-pH 9.5, and the buffer used may be, for example, Tris/hydrochloride buffer, Tris/acetate buffer, phosphate buffer, carbonate buffer, borate buffer or the like. Methods for extraction of RNA and methods for extraction of genomic DNA from plants are suited for more efficient extraction of the hop latent virus RNA from the specimen, and such methods are described in the genetic engineering protocols found in, for example, Molecular cloning (Maniatis et al., 1989, Cold Spring Harbor Laboratory Press). Extraction buffer containing guanidine thiocyanate is more preferred for RNA extraction methods while extraction buffer containing cetyl trimethylammonium bromide (CTAB) is more preferred for genomic DNA extraction methods.

The homogenization may be accomplished in any manner that crushes the cell walls of the specimen to allow extraction of the hop latent virus RNA present in the plant cells, and for example, it may be accomplished using a Polytron homogenizer, Teflon homogenizer, mortar or the like. If the specimen is homogenized in a frozen state, it will be possible to more efficiently crush the cell walls of the specimen and more easily extract the hop latent virus RNA present in the cells. Also, when RNA extraction buffer containing guanidine thiocyanate or genomic DNA extraction buffer containing CTAB as described above is used, the hop latent virus genomic RNA present in the cells can be extracted simply by repeated stirring with a vortex mixer or the like.

The hop latent virus genomic RNA extracted in the extraction step may be precipitated by alcohol precipitation and recovered and the buffer replaced with one suitable for RT-LAMP, but when the hop latent virus RNA has been extracted in distilled water or buffer with a nearly weak alkaline pH, it may be used directly for the subsequent amplification step.

In the amplification step, two different oligonucleotides consisting of the nucleotide sequences represented by SEQ ID NO: 2 and 4 of the Sequence Listing are used as primers for reverse transcription reaction using the hop latent virus RNA extracted in the extraction step as template, and then a primer set containing 4 different oligonucleotides consisting of the nucleotide sequences represented by SEQ ID NO: 1-4 of the Sequence Listing is used for repeated isothermal gene amplification reaction by Loop-mediated isothermal AMPlification (LAMP) using the cDNA obtained in the reverse transcription reaction as template.

The method for amplification of cDNA in the amplification step is known as RT-LAMP, and it can amplify a partial sequence of cDNA in the target region of hop latent virus genomic RNA by simultaneously accomplishing reverse transcription in which cDNA is synthesized by RNA, and isothermal gene amplification by LAMP, at constant temperature.

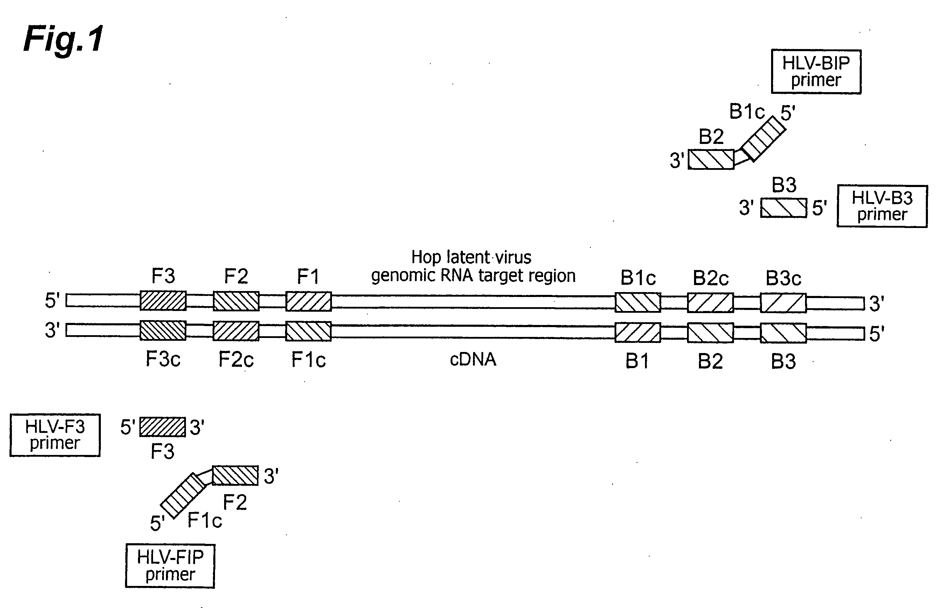

Fig. 1 is a diagram showing the positional relationship between the primer set comprising 4 different oligonucleotides consisting of the nucleotide sequences represented by SEQ ID NO: 1-4 of the Sequence Listing, and the target region of hop latent virus genomic RNA.

SEQ ID NO: 7 of the Sequence Listing represents a nucleotide sequence of cDNA in the target region of hop latent virus genomic RNA.

The oligonucleotide consisting of the nucleotide sequence represented by SEQ ID NO: 1 of the Sequence Listing (HLV-FIP primer) is an FIP primer, and it may be designed so that it carries the F2 region which is the sequence complementary to the F2c region at the 3'-end and the same sequence as the F1c region at the 5'-end.

The oligonucleotide consisting of the nucleotide sequence represented by SEQ ID NO: 2 of the Sequence Listing (HLV-BIP primer) is a BIP primer, and it may be designed so that it carries the B2 region which is the sequence complementary to the B2c region at the 3'-end and the same sequence as the B1c region at the 5'-end.

The oligonucleotide consisting of the nucleotide sequence represented by SEQ ID NO: 3 of the Sequence Listing (HLV-F3 primer) is an F3 primer, and it may be designed so that it carries the F3 region which is the sequence complementary to the F3c region.

The oligonucleotide consisting of the nucleotide sequence represented by SEQ ID NO: 4 of the Sequence Listing (HI.,V-B3 primer) is a B3 primer, and it may be designed so that it carries the B3 region which is the sequence complementary to the B3c region.

In the cDNA amplification reaction by RT-LAN1P, a reaction mixture containing the hop latent virus RNA extracted in the extraction step, a primer set containing 4 different oligonucleotides consisting of the nucleotide sequences represented by SEQ ID NO: 1-4 of the Sequence Listing, reverse transcriptase, strand displacing DNA synthase, dNTPs (dATP, dTTP, dGTP and dCTP) and buffer is allowed to stand for a prescribed time period at a constant temperature.

The temperature is preferably between 50°C and 75°C, more preferably between 60°C and 65°C, and even more preferably 65°C. A stationing time of at least 15 minutes will allow detection of cDNA amplification, but it is preferably between 15 minutes and 1 hour, and more preferably between 20 minutes and 40 minutes.

The reverse transcriptase, strand displacing DNA synthase, dNTPs and buffer may be reagents included in, for example, a Loopamp RNA Amplification Reagent Kit (product of Eiken Chemical Co., Ltd.).

In the amplification step, it is preferred to use a primer set with 6 different oligonucleotides consisting of the nucleotide sequences represented by SEQ ID NO: 1-6 of the Sequence Listing which is obtained by further adding 2 different oligonucleotide primers consisting of the nucleotide sequences represented by SEQ ID NO: 5 and 6 of the Sequence Listing, instead of a primer set containing 4 different oligonucleotides consisting of the nucleotide sequences represented by SEQ ID NO: 1-4 of the Sequence Listing.

The oligonucleotide consisting of the nucleotide sequence represented by SEQ ID NO: 5 of the Sequence Listing (HLV-LoopF primer) and the oligonucleotide consisting of the nucleotide sequence represented by SEQ ID NO: 6 of the Sequence Listing (HLV-LoopB primer) are Loop primers having sequences complementary to the single-stranded portion of the 5'-end loops of the dumbbell structure serving as the origins in the amplification reaction. HLV LoopF may be designed so as to have the sequence complementary to the sequence between the F1 region and F2 region in Fig. 1, and HLV-LoopB may be designed so as to have the sequence complementary to the sequence between the B1 region and B2 region in Fig. 1. By using HLV-LoopF and HLV-LoopB, it is possible to increase the number of origins for DNA synthesis, thereby increasing amplification efficiency and allowing the time required for amplification to be shortened to 1/3-1/2.

In the judging step, hop latent virus may be judged to be present if amplification of cDNA containing repeats of the nucleotide sequence represented by SEQ ID NO: 8 of the Sequence Listing occurs in the amplification step.

The nucleotide sequence represented by SEQ ID NO: 8 of the Sequence Listing is cDNA comprising a partial sequence of the target region of hop latent virus genomic RNA, and it is the core sequence that is repeatedly amplified in RT-LAMP using the aforementioned primer set. The nucleotide sequence of genomic RNA of hop latent virus complementary to this core sequence, and the nucleotide sequences of genomic RNA of hop latent virus to which the 4 different oligonucleotides consisting of the nucleotide sequences represented by SEQ ID NO: 1-4 of the Sequence Listing anneal, are partial sequences of the gene for the coat protein of hop latent virus, and the regions of the genomic RNA of hop latent virus containing these nucleotide sequences are suitable target regions for amplification of cDNA by RT-LAMP. Consequently, the presence of hop latent virus can be specifically detected by confirming whether or not amplified cDNA is present.

The presence of hop latent virus may be judged by, for example, unaided visual observation of the reaction mixture after amplification of the cDNA by RT-LAMP, determining hop latent virus to be present if opacity of the reaction mixture is confirmed. Alternatively, a fluorescent intercalator may be added to the reaction mixture and hop latent virus judged to be present if UV irradiation produces luminescence, indicating amplification of the cDNA.

In order to judge the presence of hop latent virus more accurately, for example, the amplified cDNA may be digested with restriction enzyme NaeI having a site in the nucleotide sequence represented by SEQ ID NO: 8 of the Sequence Listing, and then electrophoresis performed, judging hop latent virus to be present if a 285 bp DNA fragment is confirmed.

The detection primer set for hop latent virus according to the invention is characterized by comprising 4 different oligonucleotides consisting of the nucleotide sequences represented by SEQ ID NO: 1-4 of the Sequence Listing or by comprising 6 different oligonucleotides consisting of the nucleotide sequences represented by SEQ ID NO: 1-6 of the Sequence Listing.

These primer sets can be synthesized with a DNA synthesizer based on the nucleotide sequence information of SEQ ID NO: 1-6 of the Sequence Listing.

The reagents necessary for the detection method for hop latent virus according to the invention may be pre-packaged as a kit. Specifically, a kit may be provided containing the primer set comprising 4 different oligonucleotides consisting of the nucleotide sequence represented by SEQ ID NO: 1-4 of the Sequence Listing or 6 different oligonucleotides consisting of the nucleotide sequence represented by SEQ ID NO: 1-6 of the Sequence Listing, the 4 different dNTPs (dATP, dCTP, dGTP and dTTP) that are substrates for nucleic acid synthesis, reverse transcriptase that synthesizes cDNA from RNA, strand displacing DNA synthase with strand displacing activity, buffer that creates the conditions suitable for the enzyme reaction, salts (magnesium salt or manganese salt, for example) as cofactors, protective agents to stabilize the enzyme or template, and reagents required for detection of the reaction product as necessary.

The kit may further contain a positive control to confirm normal progression of RT-LAMP reaction with the primers of the invention. The positive control may be, for example, DNA containing a region amplified by the primer of the invention.

### Examples

The present invention will now be explained in greater detail using examples, with the understanding that the invention is not limited to these examples.

### (Example 1) Detection of hop latent virus by RT-LAMP

### 1) Preparation of hop tissue samples for use in detection of hop latent virus

The specimen for detection of hop latent virus was obtained by removing the roots from hop test tube seedlings grown from suckers of hops infected with hop latent virus, while the control specimen was obtained by removing the roots from virus-free hop test tube seedlings, with hop tissue samples being prepared from each specimen.

The hop latent virus detection specimen and the control specimen were each frozen in liquid nitrogen and pulverized with a mortar until the tissue became powdery, and then a 5-fold amount of distilled water by weight was added to the specimen for suspension. The hop latent virus detection specimen and control specimen suspensions obtained in this manner were used as hop tissue samples for the following experiment.

### 2) Preparation of RNA samples

To each hop tissue sample (40 µL each) there was added 160 µL each of 2 × CTAB solution (2% cetyltrimethylammonium bromide, 20 mM EDTA, 1.4 M NaCl, 5% β-mercaptoethanol, 0.1 M Tris, pH 9.5), and the mixture was warmed at 65°C for 20 minutes. It was then centrifuged at 15,000 rpm for 10 minutes to produce a residue which was removed, and the supernatant was transferred to a new Eppendorf tube. Next, 200 µL of isopropanol was added and mixed therewith and the precipitate resulting from centrifugal separation at 15,000 rpm for 10 minutes was recovered as RNA, and after rinsing with 70% ethanol and drying, it was dissolved in 40 µL of sterilized water to obtain an RNA sample.

The RNA sample of the hop latent virus detection specimen and the RNA of the control specimen were each serially diluted 10³-, 10⁴-, 10⁵-, 10⁶- and 10⁷-fold using sterilized water, and 2 µL thereof was provided as RNA-diluted sample for cDNA amplification by RT-LAMP. The RNA-diluted samples corresponded to 1/(2 × 10⁴), 1/(2 × 10⁵), 1/(2 × 10⁶), 1/(2 × 10⁷) and 1/(2 ×10⁸) of the hop tissue samples.

### 3) Primers used for RT-LAMP

For the primers there were used HLV-FIP primer (SEQ ID NO: 1) as the FIP primer, HLV-BIP primer (SEQ ID NO: 2) as the BIP primer, HLV-F3 primer (SEQ ID NO: 3) as the F3 primer, HLV-B3 primer (SEQ ID NO: 4) as the B3 primer and HLV-LoopF (SEQ ID NO: 5) and HLV-LoopB (SEQ ID NO: 6) as the Loop primers. The 6 different primers were used for amplification of cDNA by RT-LAMP, thus allowing amplification of cDNA containing repeats of the nucleotide sequence represented by SEQ ID NO: 8 of the Sequence Listing, as a partial sequence of the cDNA of the hop latent virus coat protein gene.

### 4) Amplification of cDNA by RT-LAMP

Amplification of the cDNA by RT LAMP was accomplished using a Loopamp RNA Amplification Reagent Kit (product of Eiken Chemical Co., Ltd.). Specifically, following the manufacturer's protocol, each RNA-diluted sample obtained by serial dilution (2 µl) was added to the reaction mixture, and then the 6 different primers, reverse transcriptase, strand displacing DNA synthase, dNTPs and buffer were added and the mixture was allowed to stand at 65°C for 50 minutes.

### 5) Detection of amplified cDNA

Since amplification of cDNA by RT-LAMP leads to reaction of free pyrophosphoric acid with magnesium ion to form magnesium pyrophosphate, the cDNA amplification can be determined based on the opacity of the reaction mixture. The cDNA amplified by the RT-LAMP cDNA amplification is the nucleotide sequence represented by SEQ ID NO: 8 of the Sequence Listing which corresponds to a partial sequence of the cDNA of the hop latent virus coat protein gene, and therefore opacity of the reaction mixture was judged as the presence of hop latent virus.

Table 1 shows the results of detecting hop latent virus by RT-LAMP using the RNA-diluted samples of the hop latent virus detection specimen and the RNA-diluted samples of the control specimen.

**[Table 1]**

| Degree of dilution from hop tissue sample | 2 × 10⁴ | 2 × 10⁵ | 2 ×10⁶ | 2 × 10⁷ | 2 × 10⁸ |
|---|---|---|---|---|---|
| Hop latent virus detection specimen | Opaque | Opaque | Opaque | Opaque | ND |
| Control specimen | ND | ND | ND | ND | ND |

| | | | | | |
|---|---|---|---|---|---|
| ND: No opacity observed. | | | | | |

No hop latent virus was detected in any of the RNA-diluted samples of the control specimen, but hop latent virus was detected in the RNA-diluted samples of the hop latent virus detection specimen even with a hop tissue sample at 2 × 10⁷-fold dilution. This demonstrated that when detection is carried out by RT-LAMP, hop latent virus is detected only in the specimens for hop latent virus detection which were infected with hop latent virus, and the detection sensitivity was found to be much higher than detection by ELISA, described hereunder.

Nevertheless, since opacity of the reaction mixture was observed even with nonspecific amplification of cDNA by RT-LAMP, a confirmation test was carried out by the following experiment. Specifically, 1 µL of each reaction mixture for which cDNA amplification was detected in the cDNA amplification by RT-LAMP using the RNA-diluted sample of the hop latent virus detection specimen, was digested with restriction enzyme NaeI and fractionated by polyacrylamide gel electrophoresis, and it was confirmed whether or not a 285 bp DNA fragment was detected. A NaeI site is present in the nucleotide sequence represented by SEQ ID NO: 8 of the Sequence Listing, and therefore digestion with restriction enzyme NaeI allows the cDNA amplified by RT-LAMP to be detected as a 285 bp DNA fragment. The electrophoresed gel may be dipped in ethidium bromide solution for 10 minutes to stain the fractionated DNA fragments, and then irradiated with ultraviolet rays for visualization of the DNA bands.

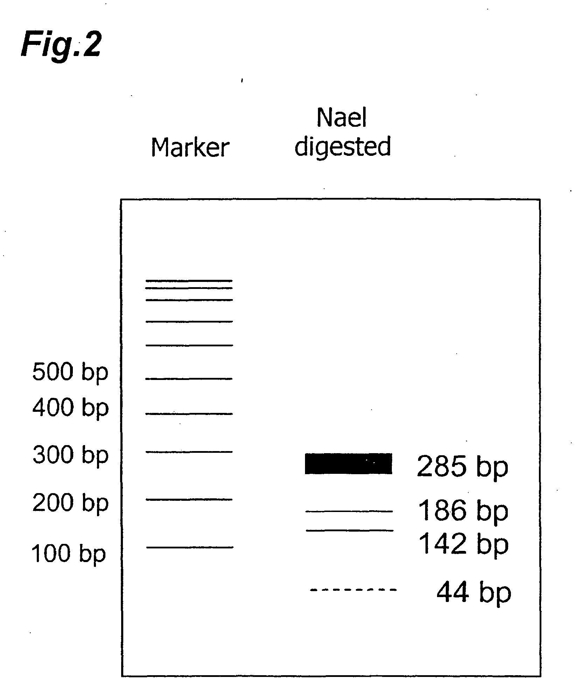

Fig. 2 is a gel diagram showing the band pattern obtained by using restriction enzyme NaeI for digestion of cDNA amplified by RT-LAMP using an RNA-diluted sample of a hop latent virus detection specimen, followed by polyacrylamide gel electrophoresis.

As a result, opacity was observed in the cDNA amplification reaction by RT-LAMP, and a 285 bp DNA fragment band was detected by digestion with restriction enzyme NaeI, for all of the cDNA in each reaction mixture in which hop latent virus was detected in Table 1. Minor bands of 186 bp and 142 bp are also seen in the gel in addition to the major band of 285 bp, but since linked cDNA partial sequences of the target region are amplified in cDNA amplification by LAMP, the presence of the minor bands of 186 bp and 142 bp in addition to the major band of 285 bp may be considered a proper result.

Thus, it was demonstrated that when opacity indicating amplification of cDNA by RT-LAMP was observed in Example 1, it was not nonspecific cDNA amplification but rather specific amplification of a portion of the hop latent virus coat protein gene, showing that the detection of hop latent virus was clearly dependent on the presence of hop latent virus.

This suggested that the detection method for hop latent virus by RT-LAMP described in Example 1 is a very highly sensitive method compared to the detection of hop latent virus by ELISA described hereunder, and that it allows the presence of hop latent virus to be easily judged merely by visually determining the opacity of the reaction mixture.

### (Comparative Example 1) Detection of hop latent virus by ELISA

### 1) Preparation of ELISA plate for detection of hop latent virus

Anti-hop latent virus antibody (provided by Plant Virology Laboratory, Faculty of Agriculture, Hokkaido University) that specifically recognizes hop latent virus was diluted to 1 µg/mL with 0.05 M SCB buffer (1.59 g/L Na₂CO₃, 2.93 g/L NaHCO₃, pH 9.6) and then dispensed into a 96-well microplate at 0.2 mL each and stationed at 37°C for 4 hours. Next, 0.02 M PBS-T (8.0 g/L NaCl, 0.2 g/L KH₂PO₄, 2.9 g/L Na₂HPO₄ · 12H₂O, 0.2 g/L KCl, 0.5 mL/L Tween-20, pH 7.4) was used for rinsing 5 times at 5 minute intervals, and the obtained antibody solid phase plate was used as an ELISA plate for detection of hop latent virus, in the following test.

### 2) Detection by ELISA

To hop tissue samples (40 µL each) prepared from the hop latent virus detection specimens and control specimens mentioned in Example 1 there was added 160 µL of distilled water, after which 200 µL of PBS-T/PVP solution (16.0 g/L NaCl, 0.4 g/L KH₂PO₄, 5.8 g/L Na₂HPO₄ · 12H₂O, 0.4 g/L KCl, 1.0 mL/L Tween-20, 40 g/L polyvinylpyrrolidone, pH 7.4) was further added and mixed therewith, the mixture was centrifuged at 15,000 rpm for 10 minutes and the supernatant was recovered for use as a protein sample (200 µL).

The hop latent virus detection specimen and control specimen protein samples were each serially diluted 10-, 10²- and 10³-fold with PBS-T/PVP solution and 200 µL of each was provided as a protein-diluted sample for ELISA. The non-diluted protein sample corresponded to 1/2 of the hop tissue sample and the protein-diluted samples serially diluted 10-, 10²- and 10³-fold corresponded respectively to 1/20, 1/(2 × 10²) and 1/(2 × 10³) of the hop tissue sample.

The protein sample and each of the protein-diluted samples (200 µL each) were added to the aforementioned ELISA plate and stationed overnight. After using 0.02 M PBS-T for rinsing 5 times at 5 minute intervals, 200 µL of alkaline phosphatase-labeled anti-hop latent virus antibody diluted 1000-fold with 0.02 M PBS-T was added to each and the mixtures were further stationed at 37°C for 3 hours. Next, each well of the ELISA plate was rinsed 5 times at 5 minute intervals using 0.02 M PBS-T, and then 200 µL of substrate solution (10% diethanolamine, 1 g/L disodium p-nitrophenylphosphate) was added and reaction was conducted for a fixed time at room temperature, after which the absorbance at 405 nm was measured.

Table 2 shows the results of detecting hop latent virus by ELISA using the protein sample and each protein-diluted sample of the hop latent virus detection specimen, and the protein sample and each protein-diluted sample of the control specimen.

**[Table 2]**

| Degree of dilution from hop tissue sample | 2 | 20 | 2 × 10² | 2 × 10³ |
|---|---|---|---|---|
| Hop latent virus detection specimen | Opaque | Opaque | ND | ND |
| Control specimen | ND | ND | ND | ND |

| | | | | |
|---|---|---|---|---|
| ND: No opacity observed. | | | | |

No hop latent virus was detected in any of the protein sample and each protein-diluted sample of the control specimen, but hop latent virus was detectable up to 20-fold dilution of the hop tissue sample, in the protein sample and each protein-diluted sample of the hop latent virus detection specimen. This indicated that, in ELISA, hop latent virus is detected only in hop latent virus detection specimens infected with hop latent virus and that hop latent virus can be properly detected, but that the detection sensitivity is at least 10⁶-fold (1,000,000-fold) lower compared to detection by RT-LAMP as described above.

### Industrial Applicability

According to the invention, it is possible to detect hop latent virus specifically and with high sensitivity in any season including during cultivation, harvesting and storage, regardless of the number of specimens or the location of evaluation. Moreover, the detection primer set and detection kit for hop latent virus according to the invention may be suitably used for RT-LAMP, thus allowing simple and high-sensitivity detection of hop latent virus.

## Claims

1. A detection method for hop latent virus that comprises
an extraction step in which hop latent virus RNA is extracted from a specimen,
an amplification step in which a primer set containing 4 different oligonucleotides consisting of the nucleotide sequences represented by SEQ ID NO: 1-4 of the Sequence Listing is used for amplification of cDNA by Reverse Transcription-Loop-mediated isothermal AMPlification (RT-LAMP) using the RNA as template, and
a judging step in which hop latent virus is judged to be present when amplification of cDNA containing the nucleotide sequence represented by SEQ ID NO: 8 of the Sequence Listing has occurred in the amplification step.

2. A detection method according to claim 1, wherein the amplification step is a step in which a primer set containing 6 different oligonucleotides consisting of the nucleotide sequences represented by SEQ ID NO: 1-6 of the Sequence Listing is used for amplification of cDNA by Reverse Transcription-Loop-mediated isothermal AMPlification (RT-LAMP) using the RNA as template.

3. A detection primer set for hop latent virus that comprises 4 different oligonucleotides consisting of the nucleotide sequences represented by SEQ ID NO: 1-4 of the Sequence Listing.

4. A detection primer set for hop latent virus that comprises 6 different oligonucleotides consisting of the nucleotide sequences represented by SEQ ID NO: 1-6 of the Sequence Listing.

5. A detection kit for hop latent virus which contains a primer set according to claim 3 or 4, reverse transcriptase, a strand displacing DNA synthase, dNTPs and a buffer.

## Patentansprüche

1. Nachweisverfahren für latentes Hopfenvirus, das umfasst:
einen Extraktionsschritt, in dem die latente Hopfenvirus-RNA aus einer Probe extrahiert wird;
einen Amplifikationsschritt, in dem ein Primerset, das 4 verschiedene Oligonucleotide beinhaltet, bestehend aus den Nucleotidsequenzen wie in SEQ ID NO: 1-4 des Sequenzprotokolls gezeigt, zur Amplifikation der cDNA durch Reverse Transcription-Loop-mediated isothermal AMPlification (RT-LAMP) unter Verwendung der RNA als Matrize verwendet wird; und
einen Auswertschritt, in dem latentes Hopfenvirus als vorhanden bewertet wird, wenn die Amplifikation der cDNA, enthaltend die Nucleotidsequenz wie durch SEQ ID NO: 8 des Sequenzprotokolls gezeigt, im Amplifikationsschritt eingetreten ist.

2. Nachweisverfahren nach Anspruch 1, wobei der Amplifikationsschritt ein Schritt ist, in dem ein Primerset, das 6 verschiedene Oligonucleotide beinhaltet, bestehend aus den Nucleotidsequenzen wie in SEQ ID NO: 1-6 des Sequenzprotokolls gezeigt, zur Amplifikation der cDNA durch Reverse Transcription-Loop-mediated isothermal AMPlification (RT-LAMP) unter Verwendung der RNA als Matrize verwendet wird.

3. Nachweisprimerset für latentes Hopfenvirus, das 4 verschiedene Oligonucleotide umfasst, die aus den Nucleotidsequenzen wie in SEQ ID NO: 1-4 des Sequenzprotokolls gezeigt bestehen.

4. Nachweisprimerset für latentes Hopfenvirus, das 6 verschiedene Oligonucleotide umfasst, die aus den Nucleotidsequenzen wie durch SEQ ID NO: 1-6 des Sequenzprotokolls gezeigt bestehen.

5. Nachweiskit für latentes Hopfenvirus, das einen Primerset nach Anspruch 3 oder 4, reverse Transkriptase, eine Strang-verdrängende DNA-Synthase, dNTPs und einen Puffer beinhaltet.

## Revendications

1. Procédé de détection du virus latent du houblon qui comprend
une étape d'extraction dans laquelle l'ARN du virus latent du houblon est extrait à partir d'un spécimen,
une étape d'amplification dans laquelle un ensemble d'amorces contenant 4 différents oligonucléotides consistant en les séquences nucléotidiques représentées par les SEQ ID No: 1 à 4 de la liste de séquences est utilisé pour l'amplification de l'ADNc par amplification isothermique médiée par des boucles et transcription inverse (RT-LAMP) utilisant l'ARN comme matrice, et
une étape d'appréciation dans laquelle le virus latent du houblon est considéré comme étant présent quand l'amplification de l'ADNc contenant la séquence nucléotidique représentée par la SEQ ID NO: 8 de la liste de séquences a eu lieu dans l'étape d'amplification.

2. Procédé de détection selon la revendication 1, dans laquelle l'étape d'amplification est une étape dans laquelle un ensemble d'amorces contenant 6 différents oligonucléotides consistant en les séquences nucléotidiques représentées par les SEQ ID NO: 1 à 6 de la liste de séquences est utilisé pour l'amplification de l'ADNc par amplification isothermique médiée par des boucles et transcription inverse (RT-LAMP) utilisant l'ARN en tant que matrice.

3. Ensemble d'amorces de détection du virus latent du houblon qui comprend 4 différents oligonucléotides consistant en les séquences nucléotidiques représentées par les SEQ ID NO: 1 à 4 de la liste de séquences.

4. Ensemble d'amorces de détection du virus latent du houblon qui comprend 6 différents oligonucléotides consistant en les séquences nucléotidiques représentées par les SEQ ID NO: 1 à 6 de la liste de séquences.

5. Kit de détection du virus latent du houblon qui contient un ensemble d'amorces selon la revendication 3 ou 4, une transcriptase inverse, une ADN synthase par déplacement de brin, des dNTP et un tampon.
